# EUROPEAN PATENT SPECIFICATION

(11) **EP 2 253 304 B1**
(45) Date of publication and mention of the grant of the patent: **11.11.2015**
(21) Application number: 10154292.6
(22) Date of filing: 22.02.2010
(51) Int. Cl.: A61K 8/37, A61K 8/65, A61K 8/67, A61K 8/73, A61K 8/92, A61Q 19/08, A61K 8/49

(54) **Nifedipine-based compositions for anti-wrinkle treatments**
Nifedipin enthaltende Zusammensetzung zur Anti-Faltenbehandlung
Composition contenant de la nifédipine pour le traitement des rides

(30) Priority: 23.02.2009 IT RM20090079
(43) Date of publication of application: 24.11.2010
(73) Proprietor: Antropoli, Carmine, 81043 S. Angelo in Formis (CE) (IT)
(72) Inventor: Antropoli, Carmine, 81043 S. Angelo in Formis (CE) (IT)
(74) Representative: Sarpi, Maurizio

(56) References cited:
- WO-A1-02/087543
- WO-A2-2008/018110
- US-A- 5 543 099
- US-A- 5 654 337
- US-A1- 2005 186 171

## Description

### BRIEF SUMMARY OF THE INVENTION

The present invention relates to the sector of cosmeceutics and more in particular to the topical use of a Nifedipine-based preparation for anti-wrinkle treatment.

According to the invention the local use of micronized Nifedipine in association with almond oil, isopropyl myristate, ialuronic acid, native collagen, vitamin E or tocopheryl acetate, vitamin A or retinal palmitate, on dry and clean skin, modulates the excessive stimulation of the mimetic muscles, relaxing the facial tension already after a few days and effectively reduces the depth and length of the wrinkles by means of a simultaneous filling action.

### FIELD OF THE INVENTION

By the term "cosmeceutics" is meant that sector of cosmetics-pharmaceutics that concerns the treatment, not of a disease, but of an individual's personal appearance, i.e., something that in the last century did not fall within the concept of health. Examples are baldness, wrinkles, aged skin, sun spots, and so forth. Such conditions have by now come to be considered as true diseases or at least conditions requiring treatment, as would be diabetes or ulcer.

Particularly felt is the problem of wrinkles, the appearance of which represents the sign of ageing of the skin and the photodamage caused by solar radiation.

Human skin consists of a number of layers, amongst which the epidermis and the dermis. The former performs a function of protection for the body, whereas the latter serves as a substrate for the epidermis and is responsible for the tone of the skin.

The compact structure of the dermis is due to the presence of collagen fibres and elastic fibres, the loss of which is one of the main factors of ageing. With time, for alterations of its tone and elasticity, the skin becomes no longer able to relax, and the first depressions remain and evolve progressively with ageing of the skin.

The contraction of the muscles of the face is the essential and predominant factor in the formation of wrinkles. The chronic contraction of the muscles of the face forms, over time, wrinkles that remain visible on the person's face even when the face is relaxed and, with the passage of time, the wrinkles tend to remain even at rest.

Hence, very wrinkle of the face reflects in its genesis a chronic contraction of a mimetic muscle:
glabellar wrinkles: they are generated by the contraction of the procerus and corrugator muscle of the eyebrow;
wrinkles of the forehead: they are generated by the contraction of the muscle of the forehead;
crow's feet: they are generated by the contraction of the orbicular muscle of the eyes;
peribuccal wrinkles: they are due to the contraction of the orbicular muscle of the mouth.

In the dermis and in the subcutis there occur a series of modifications consisting principally of:
reduction in the concentration of ialuronic acid, with consequent water loss in the tissues;
reduction of the production of sebum (in females) with the advance of the years, and consequent dryness of the skin;
greater production of collagen of Type III, i.e., anelastic and tougher, at the expense of that of Type I, i.e., "young" collagen, which bestows elasticity and plasticity on the tissues;
reduction of elastic fibres;
flattening of the dermo-epidermal junction, with reduction of its capacity to withstand mechanical stimuli;
reduction of the thickness of the dermis.

Added to these modifications, in the peribuccal district, are:
thinning of the lips;
muscular hypotonia, with consequent flattening of the labial line;
reduction of the volume of the maxillary and mandibular bone crests, with consequent exuberance of soft tissues in proportion to the bone support.

In addition to these alterations, there are some subjective factors that can accelerate the appearance of face wrinkles, such as cigarette smoking, exposure to sun, orthodontic problems, excessive mimetics, stress, and tiredness.

To reduce these wrinkles significantly and to regain a smoother skin, it is necessary to act on the main mechanism responsible for formation of wrinkles and favour decontraction of the skin.

Currently, the only drug approved for the treatment of glabellar wrinkles is botulinum toxin of Type A, which acts at the level of the peripheral nervous system, blocking the release of acetylcholine by the vesicles of the neuromuscular synapse, causing a blockage of muscular stimulation and hence a muscular flaccid paralysis.

The aesthetic use of botulinum toxin serum of Type A does not give rise to particular contraindications and the only known side effects are due to a reduced diffusion of the toxin to "healthy" terminals around the area to be treated, with consequent slight paralysis.

The real problem unresolved in this type of aesthetic intervention is constituted by the fact that the paralysis induced by the toxin is reversible in times that vary according to the muscle treated, the dose, the patient, and other unknown factors, so that after 2-6 months from the treatment of skeletal terminals, and after 8-12 months from the treatment of autonomic terminals, the patient must be treated again. In some cases, repeated treatments cause a slight desensitization, but the most serious problem arises following upon the treatment of large muscles, which must be treated with non-negligible amounts of neurotoxin. In these cases, there may arise an immunization with production by the patient of antibodies against the Type-A toxin with neutralizing action. Moreover, some rare subjects are resistant to Type-A botulinum neurotoxin from the start.

Today, there also exist alternatives to the reduction of wrinkles - even though their mechanism is not scientifically defined - that are used alongside botulinum injections. These substances, which are defined as "Botox-like substances", do not contain botulinum toxin in so far as this is very large and cannot be absorbed by the skin, but a particular protein (hexapeptide).

However, there exist two types of problems: difficulty in penetrating the skin barrier and capacity of acting only on the superficial muscles, the mimetic muscles, and not on large muscles. The type of skin moreover has a major effect on the results; thinner and dehydrated skins obtain more immediate effects, fatty and oily ones call for a greater number of applications before the first benefits appear because the product is absorbed less.

These products can be used as treatment complementary to infiltration of botulinum toxin in so far as the action of these cosmetics remains superficial, because they do not manage to act on the deep layers or to inhibit the physiological and pathological processes of the skin.

In the last few years, there has been introduced in anti-wrinkle treatment the use of Nifedipine as illustrated in the document No. WO 02/49603, entitled "Composition for prevention and alleviation of skin wrinkles" and in the patent application No. US 2002/0058682 A1, entitled "Treating skin wrinkles/fine lines with Calcium channel inhibitors", where it is disclosed how Nifedipine, a known calcium-antagonist, applied for topical use, is able to determine a resolution of the muscles responsible for formation of wrinkles.

Nifedipine acts, in fact, at the muscular level, blocking the calcium channels, which perform a key role in the contraction of the sarcomere.

However, side effects of Nifedipine on the skin have for some time been reported and described; see, for example, the patent application No. EP87870117,6, where it is emphasized how Nifedipine acts as vasodilator, producing reddening of the skin and generating a sensation of heat.

In a prior patent application No. WO2008/018110, the present applicant described the use of a galenic preparation containing as active principle just Nifedipine, between 0.3% and 10% of the total weight. This preparation, for topical use, has proven able to modulate the excessive stimulation of the mimetic muscles, relaxing the facial tension already after a few days, notwithstanding the fact that, with said preparation, the problems and the limits of absorption remain unresolved in so far as the preparation itself, to provide appreciable results, leads to an overdosage of the active principle, which in some cases can cause responses of hypersensitivity and side effects.

Moreover, once the muscular contraction has been interrupted, it has been found that it would be advisable to act on the wrinkle operating within it, improving the elasticity of the dermis and filling, by means of a filling action, the tissue defects that otherwise always remain visible after the treatment.

The purpose of the present invention is to overcome the drawbacks and the limits of anti-wrinkle products so far available, proposing, as an alternative to botulinum toxin and to preparations based exclusively upon Nifedipine, a new product with a base of Nifedipine in micronized form, in which in association other active principles are present that have emollient and eudermal properties and are able to act synergistically as co-adjuvants.

In accordance with the present invention, in the anti-wrinkle action, a preparation with a base of micronized Nifedipine in association with other active principles with emollient and eudermal properties that act synergistically as co-adjuvants, prevents any manifestation of side effects due to the use of preparations in which Nifedipine is the only active principle, and proves more effective in anti-wrinkle treatment as compared to the anti-wrinkle products so far available in so far as not only is the excessive stimulation of the mimetic muscles modulated but also reduced is both the depth and the length of existing wrinkles, which are "filled" and distended, bestowing upon the skin elasticity, softness and compactness, as will be illustrated hereinafter.

According to the invention, the active principles included in the formulation, in association with micronized Nifedipine (which is contained in a range comprised between 0.3% and 1% w/w), are: almond oil
isopropyl myristate
ialuronic acid
native collagen
vitamin E
vitamin A

The results obtained in clinical experimentation show that the association of the various ingredients introduced into the formulation, in adequate concentrations, bestows upon the product a high anti-wrinkle activity due to the synergistic action of the various ingredients that either alone or combined in different formulations and/or concentrations present a poor or slight anti-wrinkle activity. In particular:
- almond (Prunus amygdalus) oil can be contained in the formulation in an amount ranging from 5% to 10%, has markedly emollient and elasticizing properties, assisting and promoting regeneration of the skin; it is moreover known to be involved in the prevention and reduction of vibices;
- isopropyl myristate can be contained in the formulation in an amount ranging from 8% to 15%, has eudermal and emollient properties and functions as carrier of the various active ingredients, including Nifedipine, facilitating its passage through the skin barrier;
- ialuronic acid, or sodium ialuronate, which can be contained in the formulation in an amount ranging from 0.1 % to 0.5%, is a fundamental component of the dermis; thanks to its properties, it bestows upon the skin elasticity and softness and has the capacity of penetrating the layers of the epidermis and of acting within the skin for "filling" and distending wrinkles; by participating in the formation of collagen and connective tissue, ialuronic acid protects the organism from viruses and bacteria, increases the plasticity of the tissues, and guarantees the optimal hydration of the skin; it moreover has cicatricial and antiinflammatory properties;
- native collagen, in a 1% solution, which can be contained in the formulation in an amount ranging from 1% to 3%, is used in cosmetics as filling material for correcting unsightly marks, scars, and wrinkles;
- vitamin E, or tocopheryl acetate, which can be contained in the formulation in an amount ranging from 0.5% to 2%, is used in many cosmetic products thanks to its hydrating power and to its ease of absorption; its capacity for softening and protecting the skin against the dehydrating effect of sun, wind, and pollution render it particularly useful in preparation for sun and after-sun creams; furthermore, vitamin E is used as antioxidant, in order to provide protection from the damage caused by the excess of free radicals; and
- vitamin A, or retinyl palmitate, can be contained in the formulation in an amount ranging from 0.010% to 0.25%; vitamin A stimulates cell renewal and helps the skin to remain soft and hydrated.

All these active principles act together in combination in improving the anti-wrinkle action of a galenic preparation based upon Nifedipine alone, enhancing in particular its effectiveness and capacity of absorption, preventing the manifestation of side effects such as reddening and hypersensitivity of the skin and cooperating in reducing the traces of wrinkles, thus rendering the skin smooth; moreover,. According to the invention, it is important to note how the absorption by topical route at the level of the skin is positively affected also by the reduction of the dimensions of the particles of Nifedipine that is previously micronized by means of techniques of a known type.

Described hereinafter is the preparation of a formulation for a cream, comprising the active principles of the invention: this example is provided only for illustrative purposes, and the present invention is not to be considered limited by said example, which regards only a preferred formulation.

### Formulation of a cream:

| **Name** | **CAS Number** | **EINECS/ELINCS** | **% w/w** |
|---|---|---|---|
| Water | 7732-18-5 | 231-791-2 | 66.735 |
| Isopropyl myristate | 110-27-0 | 203-751-4 | 10.0 |
| Almond oil | 8007-69-0 | | 6.0 |
| Cetylstearylic alcohol | 67762-27-0 | 267-008-6 | 6.0 |
| Cetomacrogol 1000 | 68439-49-6 | | 3.0 |
| Glyceryl stearate | | | 3.0 |
| Vit. E acetate (liquid) | 7695-91-2 | 231-710-0 | 1.0 |
| Vit. A palmitate (liquid) | 79-81-2 | 201-228-5 | 0.015 |
| Lactic acid | 50-21-5 | 200-018-0 | 0.1 |
| Propyl p-oxybenzoate | 94-13-3 | 202-307-7 | 0.06 |
| Sodium ialuronate | 9067-32-7 | | 0.3 |
| 1% soluble collagen | 9007-34-5 | 232-697-4 | 2.0 |
| Sod. methyl p-oxybenz. | 5026-62-0 | 225-714-1 | 0.14 |
| Phenoxy ethanol | 122-99-6 | 204-589-7 | 0.2 |
| Imidazolidinyl urea | 39236-46-9 | 254-372-6 | 0.25 |
| Micronized Nifedipine | 21829-25-4 | | 0.5 |
| Silicone oil | 9006-65-9 | | 0.5 |
| Nile perfume | | | 0.1 |

The effectiveness, the safety and tolerability of said cream in the treatment of females with peri-ocular wrinkles has been evaluated in a non-controlled, open clinical study.

At the basal examination, all the subjects were asked to apply the cream referred to above, once a day, preferably in the evening, on the area affected to peri-ocular wrinkles. The cream was left to act for approximately 5 minutes, avoiding contact with the mucosae and the eyes, and then made to absorb completely by means of a light rubbing.

In order to investigate objectively the real effect of the active principles of the invention in promoting the reduction of wrinkles, different parameters were analysed:
photographs of selected areas of the skin with peri-orbital wrinkles;
dynamic length and depth of peri-orbital wrinkles using profilometric measurements;
level of hydration of the selected areas of the skin with peri-orbital wrinkles through corneometry;
passive transepidermal water loss (TEWL) of peri-orbital dynamic wrinkles.

TEWL, which quantifies the continuous evaporation of the skin, was measured with an evaporimeter. The value of TEWL was considered as reaching a plateau of equilibrium after 60 seconds of measurement detected by the instrument.

The medical evaluation was carried out on 64 female patients, with an average age of 43.7 years, showing the real effect of the formulation referred to above in an analysis protracted in time: it has been demonstrated that the alleviation and reduction of wrinkles is a result that can be obtained in a short period and that increases in time in the presence of a concentration of 0.5% micronized Nifedipine, which is close to the lower threshold of the range previously indicated of 0.3% to 1%.

In fact, after 45 days, the 57.4% of the persons tested presented good and excellent results: said value increased up to a final value of 78.8% after 90 days had elapsed from the first application.

In particular, it is important to emphasize how this preparation acts in the reduction of the depth of the wrinkles. This is an effect of considerable and noteworthy degree, validated by means of objective analyses (measurement of the length and depth of the wrinkles, degree of hydration of the skin, and transepidermal water loss).

The characteristics regarding the depth and length of the wrinkles and the hydration and passive transepidermal water loss of the patients enrolled appear in the table given hereinafter.

| **TABLE 1** | | | | |
|---|---|---|---|---|
| **Length** | | ***Total population*** | **ITT** | **PP** |
| | **Nbr.** | 64 | 48 | 36 |
| | **Mean** | 16.23 | 15.94 | 14.97 |
| | **Std. Deviation** | 4.55 | 4.58 | 4.32 |
| | **Median** | 15 | 15 | 15 |
| **Depth** | | ***Total population*** | **ITT** | **PP** |
| | **Nbr.** | 64 | 48 | 36 |
| | **Mean** | 7.55 | 9.03 | 10.23 |
| | **Std. Deviation** | 14.15 | 16.15 | 18.38 |
| | **Median** | 3.6 | 3.7 | 3.7 |
| **Hydration of Skin in the area of the wrinkles** | | ***Total population*** | **ITT** | **PP** |
| | **Nbr.** | 64 | 48 | 36 |
| | **Mean** | 79.41 | 83.12 | 84.85 |
| | **Std. Deviation** | 16.75 | 15.38 | 13.95 |
| | **Median** | 81.18 | 83.1 | 86.28 |
| **Passive transepidermal water loss (TEWL)** | | ***Total population*** | **ITT** | **PP** |
| | **Nbr.** | 64 | 48 | 36 |
| | **Mean** | 13.41 | 14.23 | 14.69 |
| | **Std. Deviation** | 8.28 | 9.27 | 10.51 |

Two types of analyses were conducted:
1) Intention-to-treat (ITT) analysis, considered as the main analyses, comprises all the patients with at least one value of effectiveness in the follow-up, and in this analysis the missing data was replaced by the data of the previous time;
2) Per-protocol (PP) analysis comprises all the patients that have followed the study without violations of protocol.

Of the total of 64 patients enrolled, 48 enter the ITT analysis, having at least one value detected in the follow-up.

The results of the inferential ITT and PP analyses, are given in the attached drawings, where:
Figure 1 shows a graph that gives the time trend of the mean values of the variable length of wrinkles in the intention-to-treat analysis;
Figure 2 shows a graph that gives the time trend of the mean values of the variable depth of wrinkles in the intention-to-treat analysis;
Figure 3 shows a graph that gives the time trend of the variable hydration of the skin in the intention-to-treat analysis;
Figure 4 shows a graph that gives the time trend of the mean values of the variable transepidermal water loss (TEWEL) in the intention-to-treat analysis;
Figures 5, 6, 7 and 8, show, respectively, the graphs for the time trends of the mean values of the same variables as Figures 1-4 in the per-protocol analysis.

### TIME TREND OF THE INSTRUMENTAL VARIABLES IN THE INTENTION-TO-TREAT ANALYSIS

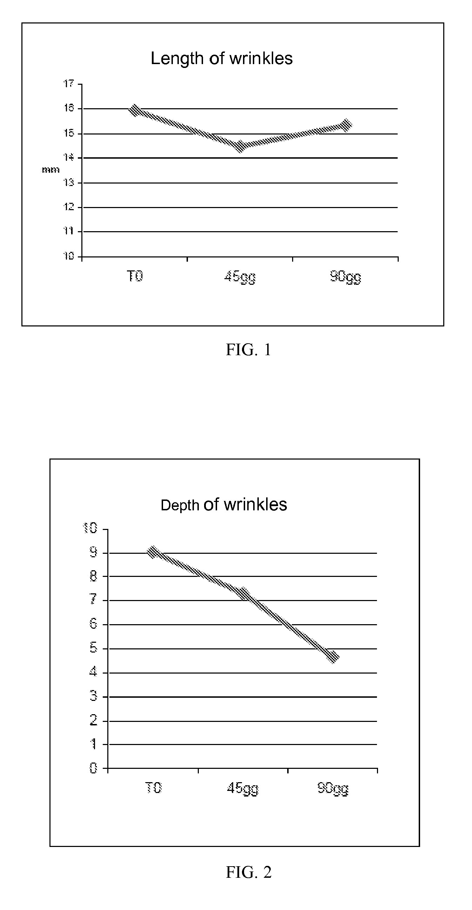

With reference to the figures, in the graph of Figure 1 regarding the time trend of the mean values of the variable length of wrinkles, there is noted a significant difference in the comparison between the time T0 and the time T45, whereas there is absence of statistical significance in the comparison between the time T45 and the time T90, even though the length of the wrinkles remains shorter than the initial value.

The graph of Figure 2 shows a significant difference, at all times, in the mean value of the variable depth of the wrinkles with respect to the basal value.

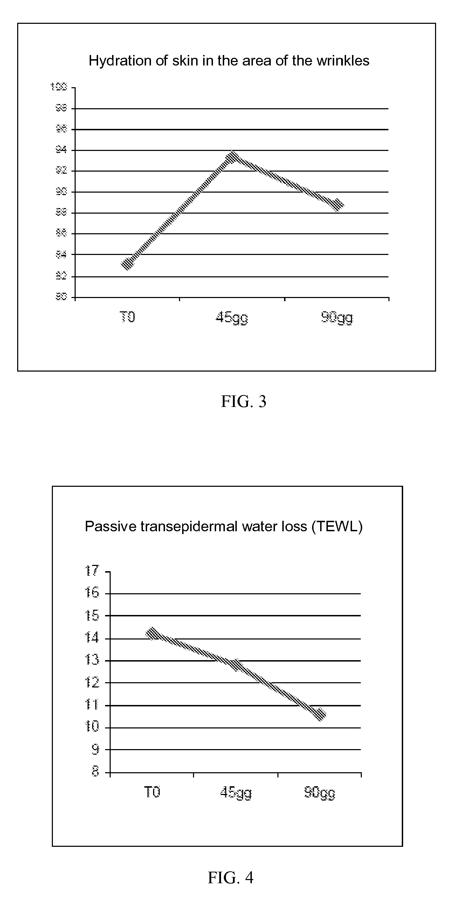

In the graph of Figure 3 there may be noted a significant difference of the mean value between the basal time and T45, whereas in the comparison between T45 and the T90 no statistically significant difference is noted, even though the hydration does not return to the initial values.

The time trend of the mean values of the variable transepidermal water loss (TEWEL) is given in the graph of Figure 4, where no significant difference is noted at any time, even though the comparison between T0 and T90 has a significance p = 0.0595.

### EFFECTIVENESS AND TOLERABILITY: TIME TREND IN ITT ANALYSIS

### Effectiveness as doctor judgement

As emerges from Table 2, the judgement of the experimenter is positive already at 45 days, when he expresses a judgement of good effectiveness for 26 patients. The judgement is of excellent effectiveness for just one patient (2.1 %), whereas for 20 patients (42.6%) the judgement is of poor effectiveness. At T90 the judgement improves sensibly in so far as for 31 patients (66%) the judgement is of good effectiveness, for 6 patients (12.8%) it is of excellent effectiveness, and for 10 patients (21.3%) it is a judgement of poor effectiveness.

### Effectiveness as patient judgement

As may be noted once again in Table 2, the patient judgement is positive already at 45 days, where the judgement is of good effectiveness for 26 patients (55.3%) and of excellent effectiveness for no patient, whereas for 21 patients (44.7%) the judgement is of poor effectiveness. At T90, the patient judgement is of good effectiveness for 32 patients (68.1 %) and of poor effectiveness for 15 patients (31.9%). The judgement does not appear to undergo any sensible modification between T45 and T90.

**TABLE 2**

| ***Effectiveness*** | ***exper_45days*** | | ***exper_90days*** | | ***pat_45days*** | | ***pat_90days*** | |
|---|---|---|---|---|---|---|---|---|
| | ***Freq*** | ***%*** | ***Freq*** | ***%*** | ***Freq*** | ***%*** | ***Freq*** | ***%*** |
| **Excellent** | 1 | 2.1 | 6 | 12.8 | 0 | 0 | 0 | 0 |
| **Good** | 26 | 55.3 | 31 | 66 | 26 | 55.3 | 32 | 68.1 |
| **Poor** | 20 | 42.6 | 10 | 21.3 | 21 | 44.7 | 15 | 31.9 |
| **Total** | 47 | 100 | 47 | 100 | 47 | 100 | 47 | 100 |
| **Effectiveness trend (experimenter)** | ***DF*** | ***Value*** | ***Prob*** | | | | | |
| | 1 | 9.9412 | 0.0016 | | | | | |
| **Effectiveness trend (patient)** | ***DF*** | ***Value*** | ***Prob*** | | | | | |
| | 1 | 3 | 0.0833 | | | | | |

### Tolerability as doctor judgement

From Table 3 it emerges that the judgement regarding tolerability expressed by the experimenter is one of excellent tolerability for 42 patients (89.4%) at T45 and for 45 patients (95.7%) at T90. Good tolerability was found in 5 patients (10.6%) at T45 and in 2 patients (4.3%) at T90. No judgement of poor tolerability emerged.

The judgement of tolerability expressed by the experimenter remains not significantly different over time.

**TABLE 3**

| ***tolerability*** | ***exper_45days*** | | ***exper_90days*** | | ***pat_45days*** | | ***pat_90days*** | |
|---|---|---|---|---|---|---|---|---|
| | ***Freq*** | ***%*** | ***Freq*** | ***%*** | ***Freq*** | ***%*** | ***Freq*** | ***%*** |
| **Excellent** | 42 | 89.4 | 45 | 95.7 | 42 | 89.4 | 45 | 95.7 |
| **Good** | 5 | 10.6 | 2 | 4.3 | 4 | 8.5 | 2 | 4.3 |
| **Poor** | 0 | 0 | 0 | 0 | 1 | 2.1 | 0 | 0 |
| **Total** | 47 | 100 | 47 | 100 | 47 | 100 | 47 | 100 |
| **Tolerability trend (experimenter)** | ***DF*** | ***Value*** | ***Prob*** | | | | | |
| | 1 | 1.8 | 0.1797 | | | | | |
| **Tolerability trend (patient)** | ***DF*** | ***Value*** | ***Prob*** | | | | | |
| | 1 | 4 | 0.0455 | | | | | |

### Tolerability as patient judgement

From the same Table 3 it emerges that the judgement regarding the tolerability expressed by the patient is excellent in the case of 42 patients (89%) at T45 and of 45 patients (95.7%) at T90. Good tolerability was found in 4 patients (8.5%) at T45 and in 2 patients (4.3%) at T90.

Just one judgement of poor tolerability was found at T45.

### TIME TREND OF THE INSTRUMENTAL VARIABLES IN PER-PROTOCOL ANALYSIS

### Variable: length of wrinkles

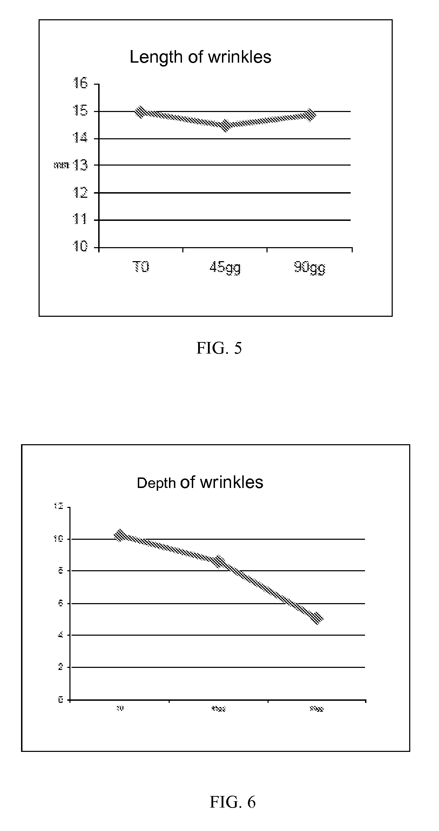

In the graph of Figure 5, no significant difference was noted in the comparison between the time T0 and the times T45 and T90; however, a slight improvement was found, even though without statistical significance, between the times T0 and T45.

### Variable: depth of wrinkles

The time trend of the mean values is given in the graph of Figure 6. There is noted a significant difference at all the times envisaged by the study protocol with respect to the basal value.

### Variable: hydration of the skin

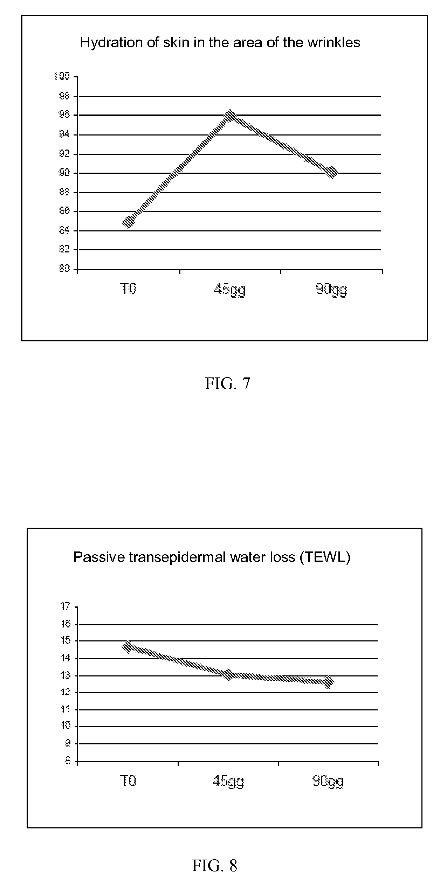

In the graph of Figure 7 there is noted a significant difference between the basal time and T45, whereas no significant difference is found at the other times analysed, even though the final mean value does not return to the initial one.

### Variable: passive transepidermal water loss

The time trend of the mean values is given in the graph of Figure 8. No significant difference is noted at any time, even though there is a reduction in transepidermal loss with respect to the basal value.

### EFFECTIVENESS AND TOLERABLITY: TIME TREND IN PER-PROTOCOL ANALYSIS

### Experimenter assessment of effectiveness

As emerges from Table 4, the judgement of the experimenter was one of poor effectiveness after 45 days of treatment for 15 patients (42.9%), one of good effectiveness at T45 for 20 patients (57.1 %), and one of excellent effectiveness for no patient. After 90 days, the assessment made by the experimenter shows an improvement in so far as the judgement was one of poor effectiveness in 5 cases (14.3%), good effectiveness in 25 cases (71.4%), and excellent effectiveness in 5 cases (14.3). Consequently, after 90 days of treatment a statistically significant value emerged.

**TABLE 4**

| ***effectiveness*** | ***Exper_45days*** | | ***Exper_90days*** | |
|---|---|---|---|---|
| | ***Freq*** | ***%*** | ***Freq*** | ***%*** |
| **Excellent** | 0 | 0 | 5 | 14.3 |
| | 20 | 57.1 | 25 | 71.4 |
| **Poor** | 15 | 42.9 | 5 | 14.3 |
| **Absent** | 0 | 0 | 0 | 0 |
| **Total** | 35 | 100 | 35 | 100 |
| **Effectiveness trend** | ***DF*** | ***Value*** | ***Prob*** | |
| | 1 | 9.9412 | 0.0016 | |

| | | | | |
|---|---|---|---|---|
| Patient assessment of effectiveness | | | | |

As emerges from Table 5, after 45 days of treatment the patient judgement was one of poor effectiveness in 18 cases (50%), good effectiveness in the remaining 18 cases (50%), and there was no judgement of excellent effectiveness. The trend shows an improvement at T90, where there remain 12 patients that express a judgement of poor effectiveness (33.3%), and 24 patients that express a judgement of good effectiveness (66.7%); once again at this time there is no judgement of excellent effectiveness.

**TABLE 5**

| ***effectiveness*** | ***pat_45days*** | | ***pat_90days*** | |
|---|---|---|---|---|
| | ***Freq*** | ***%*** | ***Freq*** | ***%*** |
| **Excellent** | 0 | 0 | 0 | 0 |
| **Good** | 18 | 50 | 24 | 66.7 |
| **Poor** | 18 | 50 | 12 | 33.3 |
| **Absent** | 0 | 0 | 0 | 0 |
| **Total** | 36 | 100 | 36 | 100 |
| **Effectiveness trend** | ***DF*** | ***Value*** | ***Prob*** | |
| | 1 | 3 | 0.0833 | |

### Tolerability as doctor judgement

From Table 6 it emerges that the judgement regarding the tolerability expressed by the experimenter at T45 is one of excellent tolerability in 32 patients (88.9%), and good tolerability in 4 patients (11.1%). After 90 days the judgement was one of excellent tolerability for 35 patients (97.2%), and of good tolerability for just one patient (1.8%). No judgement of poor tolerability was expressed at any time. The judgement of tolerability remains not significantly different over time.

**TABLE 6**

| ***tolerability*** | ***exper_45days*** | | ***exper_90days*** | |
|---|---|---|---|---|
| | ***Freq*** | ***%*** | ***Freq*** | ***%*** |
| **Excellent** | 32 | 88.9 | 35 | 97.2 |
| **Good** | 4 | 11.1 | 1 | 2.8 |
| **Total** | 36 | 100 | 36 | 100 |
| | ***DF*** | ***Value*** | ***Prob*** | |
| **Tolerability trend** | 1 | 1.8 | 0.1797 | |

### Tolerability as patient judgement

From Table 7 it emerges that the patient judgement regarding tolerability is one of excellent tolerability in 32 patients (88.9%) at T45 and in 35 patients at T90. Good tolerability was expressed in the case of 3 patients (8.3%) at T45, and in the case of 1 patient (2.8%) at T90; just one patient expressed a judgement of poor tolerability (2.8%) after 45 days of treatment.

**TABLE 7**

| ***tolerability*** | ***pat_45days*** | | ***pat_90days*** | |
|---|---|---|---|---|
| | ***Freq*** | ***%*** | ***Freq*** | ***%*** |
| **Excellent** | 32 | 88.9 | 35 | 97.2 |
| **Good** | 3 | 8.3 | 1 | 2.8 |
| **Poor** | 1 | 2.8 | 0 | 0 |
| **Total** | 36 | 100 | 36 | 100 |
| **Tolerability trend** | ***DF*** | ***Value*** | ***Prob*** | |
| | 1 | 4 | 0.0455 | |

The trend of tolerability according to patient judgement appears to undergo a positive modification between T45 and T90, coinciding with the doctor judgement.

From the data gathered, it is hence evident how the topical use of a cream containing, as active principles, Nifedipine (preferably micronized) in association with almond oil, isopropyl myristate, ialuronic acid (sodium ialuronate), native collagen, vitamin E (tocopheryl acetate), and vitamin A (retinyl palmitate) leads to:
- a statistically significant reduction in the depth of the wrinkles at all the times contemplated by the study protocol;
- a statistically significant reduction in the length of the wrinkles in the first 45 days of treatment;

- an increase in hydration of the skin that is also significant for the first 45 days, decreasing during the subsequent 45 days, and not returning in any case to the initial values; and
- a reduction in transepidermal water loss at all times recorded.

Both the values linked to hydration of the skin and those linked to transepidermal water loss indicate that the cream forming the subject of the present invention has a good hydrating power.

Worthy of particular comment is the capacity demonstrated of reducing the depth of the wrinkles. This is a noteworthy effect both from the statistical standpoint and from the standpoint of clinical relevance. In fact, at 45 days from the first application a reduction of the depth of the wrinkles of 20% was recorded, which increased to 48.5% after 90 days. The increase in the degree of hydration found in all patients was constant.

After 2 weeks from suspension of the treatment, 95% of the patients still presented appreciable results.

The tolerability of the product was 100%.

### CONCLUSIONS

Nifedipine is a known calcium-antagonist drug used since the eighties for cardiovascular pathological conditions and only by oral route. It acts as myorelaxing agent through the inhibition of the flow of the Ca++ in the sarcoplasm of smooth muscle, with consequent oxygen saving as well as a reduction in the mechanical contraction of the muscle fibres.

The studies that have investigated the use of a preparation with a base of micronized Nifedipine with the association of various active principles - almond oil, isopropyl myristate, vitamins A and E, collagen, and ialuronic acid - having eudermal and emollient properties that act with a marked synergistic and co-adjuvant action on the tone of the mimetic muscles of the face, have demonstrated that the local use of said micronized Nifedipine-based preparation, applied every evening on dry and clean skin, modulates the excessive stimulation of the mimetic muscles, relaxing facial tension already after 6 days, with a real reduction of the depth of the wrinkles by 20% after 45 days and by 48.5% after 90 days from the first application, without developing any side effects.

In particular, from the results obtained in these studies it may be suggested that the pharmacological approach of a micronized Nifedipine-based preparation with the synergistic action of various active principles associated thereto, such as almond oil, isopropyl myristate, vitamins A and E, collagen, and ialuronic acid, would at least be able to delay the progression of wrinkles and avoid the need for recourse to costly and more invasive inoculations of botulinum toxin, with the added advantage of simplicity of use at home.

Another advantage of the anti-wrinkle preparation according to the present invention lies in the fact that, whereas Botox can be injected only in the upper part of the face, the present preparation can be spread over the entire surface of the face, without this requiring any direct intervention on the part of a doctor.

This enables intervention not only on glabellar wrinkles, as in the case of botulinum toxin, but also on all the mimetic muscles, including the orbicular muscle of the eyes, so that it constitutes a valid therapeutic instrument against the pathological condition of palpebral ptosis, which avoids the need for surgical interventions such as blepharoplasty.

## Claims

1. A preparation for topical use for anti-wrinkle treatment comprising as main active principle micronized Nifedipine, in association with almond oil, isopropyl myristate, ialuronic acid or sodium ialuronate , native collagen, vitamin E or tocopheryl acetate, and vitamin A or retinyl palmitate.

2. The preparation for topical use as per Claim 1, **characterized in that** the micronized Nifedipine is present in a percentage of between 0.3% and 1 % of its total weight.

3. The preparation for topical use according to either Claim 1 or Claim 2, **characterized in that** almond oil is present in a percentage of between 5% and 10% of its total weight.

4. The preparation for topical use according to any one of the preceding claims, **characterized in that** isopropyl myristate is present in a percentage of between 8% and 15% of its total weight.

5. The preparation for topical use according to any one of the preceding claims, **characterized in that** ialuronic acid or sodium ialuronate is present in a percentage of between 0.1% and 0.5% of its total weight.

6. The preparation for topical use according to any one of the preceding claims, **characterized in that** the native collagen (1% solution) is present in a percentage of between 1% and 3% of its total weight.

7. The preparation for topical use according to any one of the preceding claims, **characterized in that** vitamin E or tocopheryl acetate is present in a percentage of between 0.5% and 2% of its total weight.

8. The preparation for topical use according to any one of the preceding claims, **characterized in that** vitamin A or retinyl palmitate is present in a percentage of between 0.010% and 0.25% of its total weight.

9. The preparation for topical use according to any one of the preceding claims, **characterized in that** is in the form of gel, cream, ointment, emulsion or oil.

10. The preparation for topical use according to any one of the preceding claims, **characterized in that** it comprises all the ingredients referred to in Claim 1.

11. A preparation for topical use for anti-wrinkle treatment containing the following formulation, expressed in % w/w:
water (66.735), isopropyl myristate (10.0), almond oil (6.0), cetylstearylic alcohol (6.0), Cetomacrogol 1000 (3.0), glyceryl stearate (3.0), vitamin E acetate (liquid) (1.0), vitamin A palmitate (liquid) (0.015), lactic acid (0.1), propyl p-oxybenzoate (0.06), sodium ialuronate (0.3%), 1 % soluble collagen (2.0), methyl p-oxybenzoate (0.14), fenoxyethanol (0.2), imidazolidinyl urea (0.25), micronized Nifedipine (0.5), silicone oil (0.5), nile perfume (0.1).

## Patentansprüche

1. Zubereitung zur äußeren Anwendung für Anti-Faltenbehandlung, umfassend als Hauptwirkstoff mikronisiertes Nifedipin, in Verbindung mit Mandelöl, Isopropylmyristat, Hyaluronsäure oder Natriumhyaluronat, nativem Kollagen, Vitamin E oder Tocopherylacetat, und Vitamin A oder Retinylpalmitat.

2. Zubereitung zur äußeren Anwendung nach Anspruch 1,
**dadurch gekennzeichnet, dass** das mikronisierte Nifedipin darin mit einem Prozentsatz zwischen 0,3% und 1% ihres Gesamtgewichts enthalten ist.

3. Zubereitung zur äußeren Anwendung nach Anspruch 1 oder Anspruch 2,
**dadurch gekennzeichnet, dass** Mandelöl darin mit einem Prozentsatz zwischen 5% und 10% ihres Gesamtgewichts enthalten ist.

4. Zubereitung zur äußeren Anwendung nach einem der vorhergehenden Ansprüche,
**dadurch gekennzeichnet, dass** Isopropylmyristat darin mit einem Prozentsatz zwischen 8% und 15% ihres Gesamtgewichts enthalten ist.

5. Zubereitung zur äußeren Anwendung nach einem der vorhergehenden Ansprüche,
**dadurch gekennzeichnet, dass** Hyaluronsäure oder Natriumhyaluronat darin mit einem Prozentsatz zwischen 0,1% und 0,5% ihres Gesamtgewichts enthalten ist.

6. Zubereitung zur äußeren Anwendung nach einem der vorhergehenden Ansprüche,
**dadurch gekennzeichnet, dass** das native Kollagen (1%-ige Lösung) darin mit einem Prozentsatz zwischen 1% und 3% ihres Gesamtgewichts enthalten ist.

7. Zubereitung zur äußeren Anwendung nach einem der vorhergehenden Ansprüche,
**dadurch gekennzeichnet, dass** Vitamin E oder Tocopherylacetat darin mit einem Prozentsatz zwischen 0,5% und 2% ihres Gesamtgewichts enthalten ist.

8. Zubereitung zur äußeren Anwendung nach einem der vorhergehenden Ansprüche,
**dadurch gekennzeichnet, dass** Vitamin A oder Retinylpalmitat darin mit einem Prozentsatz zwischen 0,010% und 0,25% ihres Gesamtgewichts enthalten ist.

9. Zubereitung zur äußeren Anwendung nach einem der vorhergehenden Ansprüche,
**dadurch gekennzeichnet, dass** sie in Form eines Gels, einer Creme, einer Salbe, einer Emulsion oder eines Öls vorliegt.

10. Zubereitung zur äußeren Anwendung nach einem der vorhergehenden Ansprüche,
**dadurch gekennzeichnet, dass** sie all die Inhaltsstoffe umfasst, auf die in Anspruch 1 Bezug genommen wird.

11. Zubereitung zur äußeren Anwendung für Anti-Faltenbehandlung, die die nachstehende Formulierung enthält, ausgedrückt in Gewichts-%:
Wasser (66,735), Isopropylmyristat (10,0), Mandelöl (6,0),
Cetylstearylalkohol (6,0), Cetomacrogol 1000 (3,0), Glycerinstearat (3,0),
Vitamin-E-Acetat (flüssig) (1,0), Vitamin-A-Palmitat (flüssig) (0,015),
Milchsäure (0,1), Propyl-p-oxybenzoat (0,06), Natriumhyaluronat (0,3%),
1%-iges lösliches Kollagen (2,0), Methyl-p-oxybenzoat (0,14),
Phenoxyethanol (0,2), Imidazolidinylharnstoff (0,25), mikronisiertes Nifedipin (0,5), Silikonöl (0,5), Nile-Parfüm (0,1).

## Revendications

1. Préparation à usage topique pour le traitement antirides comprenant comme principe actif principal de la nifédipine micronisée, en association avec l'huile d'amande, le myristate d'isopropyle, l'acide hyaluronique ou l'hyaluronate de sodium, le collagène natif, l'acétate de tocophéryle ou vitamine E et le palmitate de rétinyle ou vitamine A.

2. Préparation à usage topique selon la revendication 1, **caractérisé en ce que** la nifédipine micronisée est présente dans un pourcentage compris entre 0,3% et 1% de son poids total.

3. Préparation à usage topique selon la revendication 1 ou la revendication 2, **caractérisé en ce que** l'huile d'amande est présente dans un pourcentage compris entre 5% et 10% de son poids total.

4. Préparation à usage topique selon l'une quelconque des revendications précédentes, **caractérisé en ce que** le myristate d'isopropyle est présent dans un pourcentage compris entre 8% et 15% de son poids total.

5. Préparation à usage topique selon l'une quelconque des revendications précédentes, **caractérisé en ce que** l'acide hyaluronique ou l'hyaluronate de sodium est présent dans un pourcentage compris entre 0,1% et 0,5% de son poids total.

6. Préparation à usage topique selon l'une quelconque des revendications précédentes, **caractérisé en ce que** le collagène natif (solution à 1%) est présent dans un pourcentage compris entre 1% et 3% de son poids total.

7. Préparation à usage topique selon l'une quelconque des revendications précédentes, **caractérisé en ce que** la vitamine E ou acétate de tocophéryle est présent dans un pourcentage compris entre 0,5% et 2% de son poids total.

8. Préparation à usage topique selon l'une quelconque des revendications précédentes, **caractérisé en ce que** la vitamine A ou palmitate de rétinyle est présent dans un pourcentage compris entre 0,010% et 0,25% de son poids total.

9. Préparation à usage topique selon l'une quelconque des revendications précédentes, **caractérisé en ce qu'**elle est sous la forme de gel, crème, onguent, émulsion ou huile.

10. Préparation à usage topique selon l'une quelconque des revendications précédentes, **caractérisé en ce qu'**elle comprend tous les ingrédients indiqués dans la revendication 1.

11. Préparation à usage topique pour le traitement antirides comprenant la formulation suivante, exprimée en % en poids :
eau (66,735), myristate d'isopropyle (10,0), huile d'amande (6,0), alcool stéarique-cétylique (6,0), Cetomacrogol 1000 (3,0), stéarate de glycéryle (3,0), acétate vitamine E (liquide) (1,0), palmitate vitamine A (liquide) (0,015), acide lactique (0,1), p-oxybenzoate de propyle (0,06), hyaluronate de sodium (0,3%), collagène soluble à 1% (2,0), p-oxybenzoate de méthyle (0,14), phénoxyéthanol (0,2), imidazolidinyl urée (0,25), nifédipine micronisée (0,5), huile de silicone (0,5), parfum du Nil (0, 1).
